Europäisches Patentamt

European Patent Office

Office européen des brevets

(19)

(11) Veröffentlichungsnummer : **0 322 660 B1**

# (12) EUROPÄISCHE PATENTSCHRIFT

(45) Veröffentlichungstag der Patentschrift :
28.08.91 Patentblatt 91/35

(51) Int. Cl.$^5$ : **C07C 47/02, C07C 45/78**

(21) Anmeldenummer : 88120964.7

(22) Anmeldetag : 15.12.88

(54) Verfahren zur Gewinnung von 2-Methylbutanal.

(30) Priorität : 24.12.87 DE 3744212

(43) Veröffentlichungstag der Anmeldung :
05.07.89 Patentblatt 89/27

(45) Bekanntmachung des Hinweises auf die
Patenterteilung :
28.08.91 Patentblatt 91/35

(84) Benannte Vertragsstaaten :
AT BE CH DE ES FR GB IT LI NL SE

(56) Entgegenhaltungen :
EP-A- 0 215 611
AT-B- 337 145
DE-A- 2 833 538
DE-B- 2 855 506
DE-C- 960 187
DE-C- 2 218 305
US-A- 4 484 006

(56) Entgegenhaltungen :
SOVIET INVENTIONS ILLUSTRATED, Sektion
Ch, Week 8345, Dec. 21, 1983; Derwent Publi-
cations, Ltd., London, E17
SOVIET INVENTIONS ILLUSTRATED, Sektion
Ch, Week K12, May 4, 1983; Derwent Publica-
tions, Ltd., London, E17

(73) Patentinhaber : HOECHST
AKTIENGESELLSCHAFT
Postfach 80 03 20
W-6230 Frankfurt am Main 80 (DE)

(72) Erfinder : Weber, Jürgen, Dr. Dipl.-Chem.
Bunsenstrasse 17
W-4200 Oberhausen 11 (DE)
Erfinder : Lappe, Peter, Dr. Dipl.-Chem.
Eickenhof 34
W-4220 Dinslaken (DE)
Erfinder : Springer, Helmut, Dipl.-Ing.
Borbecker Strasse 19
W-4200 Oberhausen 11 (DE)

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft ein Verfahren zur Gewinnung von 2-Methylbutanal aus Gemischen isomerer $C_5$-Aldehyde, insbesondere solcher, die durch Hydroformylierung von Buten-Gemischen erhalten werden.

2-Methylbutanal ist ein wertvolles Zwischenprodukt für zahlreiche technisch ausgeübte Verfahren. Es wird unter anderem durch Oxidation zu 2-Methylbuttersäure weiterverarbeitet, die z.B. als Riechstoff-Vorprodukt Verwendung findet.

2-Methylbutanal ist durch Umsetzung von n-Butyraldehyd mit Formaldehyd zugänglich. In Gegenwart alkalischer Reagenzien bildet sich aus den Ausgangsstoffen 2-Methylenbutanal, das in einer weiteren Verfahrensstufe partiell zur 2-Methylverbindung hydriert wird. Dieser Prozeß liefert zwar ein reines 2-Methylbutanal, der mit seiner Durchführung verbundene technische Aufwand ist aber sehr hoch. Hierbei ist zu berücksichtigen, daß die Umsetzung in zwei Stufen durchgeführt werden muß und die partielle Hydrierung den Einsatz kostenintensiver Edelmetallkatalyatoren erfordert.

Ein alternatives Verfahren zur Herstellung von 2-Methylbutanal ist die Hydroformylierung von Buten-2. Es gestaltet sich besonders wirtschaftlich, wenn nicht vom reinen Buten-2 ausgegangen, sondern die $C_4$-Fraktion der Naphthaspaltung als Einsatzmaterial verwendet wird. Diese Fraktion fällt z.B. als Nebenprodukt bei der Herstellung von Ethylen und Benzin an und enthält Butadien sowie Butan- und Butenisomere, nämlich n-Butan, i-Butan, Buten-1, Buten-2 und i-Buten.

Durch Hydroformylierung der von Butadien und i-Buten weitgehend befreiten $C_4$-Fraktion gelangt man zu einem Gemisch, das neben anderen Komponenten die $C_5$-Aldehyde n-Pentanal, 2-Methylbutanal und geringe Mengen 3-Methylbutanal enthält.

Die Trennung von Aldehydgemischen erfolgt in technischen Prozessen überwiegend durch fraktionierte Destillation. Wegen der Oxidationsempfindlichkeit der meisten Aldehyde, ihrer Neigung zu höhermolekularen Folgeprodukten zu kondensieren und sich thermisch zu zersetzen, sind bestimmte Vorsichtsmaßnahmen bei der Destillation einzuhalten. Empfindliche Aldehyde werden bei möglichst milden Temperaturen und nach vollständiger Entfernung der Katalysatoren destillativ getrennt. Darüber hinaus wendet man die Verfahren der azeotropen und der extraktiven Destillation an, um Kondensations- und Zersetzungsreaktionen zu vermeiden.

Die Komponenten des bei der Hydroformylierung der $C_4$-Fraktion erhaltenen Aldehydgemisches weisen nur wenig unterschiedliche Siedepunkte auf. Eine destillative Trennung ist daher nur mit äußerst trennstarken Kolonnen bei hohen Rücklaufverhältnissen möglich. Diese Bedingungen stehen nicht nur der Wirtschaftlichkeit des Trennverfahrens entgegen. Es können auch erhebliche Ausbeuteminderungen auftreten, verursacht durch die Bildung höhersiedender Verbindungen aus den thermisch wenig stabilen n-Aldehyden.

Da die vorstehend geschilderten Probleme allgemein auftreten, hat man sich bemüht, Verfahren zu entwickeln, die es erlauben, Aldehydgemische, wie sie z.B. bei der Hydroformylierung gebildet werden, einfach und effektiv zu trennen.

Nach der DE 2833538 C2 werden alpha-methylverzweigte Aldehyde der allgemeinen Formel R-CH (CH$_3$)-CHO, wobei R für geradkettige Alkylreste mit 7 bis 12 Kohlenstoffatomen steht, von den geradkettigen Verbindungen mit der gleichen Anzahl Kohlenstoffatome durch thermische Behandlung, die in einer Destillationskolonne erfolgen kann, abgetrennt. Hierbei destillieren die verzweigten Aldehyde ab, während die geradkettigen Aldehyde in schwerflüchtige Verbindungen übergehen und im Sumpf der Kolonne zurückbleiben. Dieses Verfahren wird bevorzugt dann angewendet, wenn die geradkettigen Aldehyde nicht verwertet werden können.

Gegenstand der DE 2459152 C2 ist ein Verfahren zur Gewinnung reiner n-Aldehyde aus Mischungen mit ihren Isomeren, insbesondere aus dem rohen, bei der Hydroformylierung endständiger Olefine anfallenden und gegebenenfalls vom Katalysator abgetrennten Reaktionsgemisch. Es besteht darin, daß das Reaktionsgemisch in Gegenwart eines Lösungsmittels mit der zur Fällung des n-Aldehyds als Hydrogensulfit-Additionsverbindung erforderlichen, stöchiometrischen oder unterstöchiometrischen Menge Alkalihydrogensulfit umgesetzt, der entstehende Niederschlag abgetrennt, gewaschen und anschließend in üblicher Weise gespalten wird. Wegen des nicht unerheblichen Chemikalienaufwandes eignet sich dieses Verfahren insbesondere zur Gewinnung wertvoller n-Aldehyde.

Ebenfalls über die Bisulfitverbindungen erfolgt die Zerlegung von Gemischen isomerer Aldehyde nach einer Arbeitsweise, die in der DE 960187 C1 beschrieben ist. Das Aldehydgemisch wird zunächst durch Behandeln mit der wäßrigen Lösung eines neutralen Sulfits und einer etwa äquivalenten Menge einer schwach sauren Verbindung in ein Gemisch der Bisulfitverbindungen übergeführt, das man sodann auf steigende Temperaturen erhitzt. Die hierbei in der Reihenfolge ihrer Siedepunkte nacheinander freiwerdenden Aldehyde zieht man getrennt ab und verwendet die zurückbleibende, abgekühlte Lösung erneut für die Herstellung der Bisulfitverbindungen. Dieser Prozeß ist nur auf die Trennung der leicht flüchtigen niederen Aldehyde anwendbar, da höhere Aldehyde sich bei ihren Siedepunkten schon merklich zersetzen und daher destillativ nicht abgetrennt werden können.

Ein Verfahren zur Reindarstellung von n-Aldehyden aus n/iso-Aldehydgemischen besteht in der Behandlung der Gemische mit nicht-oxidierenden starken Mineralsäuren (DE 2218305). Die geradkettigen Aldehyde gehen dabei in 1,3,5-Trioxane über, die durch fraktionierte Kristallisation abgetrennt werden. Anschließend depolymerisiert man die Trioxane in einer Destillationsapparatur unter Zusatz geringer Mengen Phosphor(V)-oxid. Das Verfahren beruht also auf der unterschiedlichen Kristallisationsfähigkeit und Löslichkeit der trimeren n-Alkanale und iso-Alkanale. Es ist nicht anwendbar, wenn außer den n-Alkanalen auch die iso-Alkanale kristallin sind. Die Bedeutung dieser Arbeitsweise ist daher begrenzt.

Es bestand somit die Aufgabe, ein Verfahren zur entwickeln, das es erlaubt, 2-Methylbutanal aus dem Gemisch isomerer Aldehyde, das z.B. bei der Hydroformylierung isomerer Butene anfällt, zu gewinnen.

Überraschenderweise wird diese Aufgabe gelöst durch ein Verfahren zur Gewinnung von 2-Methylbutanal aus den Gemischen der isomeren $C_5$-Aldehyde, insbesondere Gemischen, die bei der Hydroformylierung isomer Butene entstehen, das dadurch gekennzeichnet ist, daß man das $C_5$-Aldehydgemisch in Gegenwart von Formaldehyd und einem Aldolisierungskatalysator destilliert.

Es ist bekannt, daß Aldehyde, die in alpha-Stellung zur Carbonylgruppe eine Methylengruppe—$CH_2$—aufweisen, mit Formaldehyd in Gegenwart geeigneter Katalysatoren zu 2-Methylenaldehyden reagieren. Die Umsetzung erfolgt üblicherweise unter Druck und — unabhängig von der gewählten Temperatur — bei Verweilzeiten, die in der Größenordnung von wenigen Sekunden bis zu Stunden liegen.

Ähnlich reaktiv sind auch Aldehyde, die an alpha-ständigen Kohlenstoffatomen nur ein Wasserstoffatom tragen. Sie ergeben mit Formaldehyd sehr leicht die entsprechende alpha-Methylolverbindung.

Es war nicht vorauszusehen, daß es trotz der hohen Reaktivität sowohl der in alpha-Stellung unverzweigten als auch der in alpha-Stellung monoverzweigten Aldehyde möglich ist, ein Gemisch dieser beiden Isomerenarten durch gezielte Umsetzung der in alpha-Stellung unverzweigten Bestandteile in die Komponenten zu zerlegen.

Das neue Verfahren eignet sich ganz allgemein zur Gewinnung von 2-Methylbutanal aus den Gemischen isomerer $C_5$-Aldehyde, insbesondere aus Gemischen, die bei der Hydroformylierung der Buten-Isomeren anfallen.

Gemische mit hohem Anteil an $C_4$-Kohlenwasserstoffen, darunter auch Butene, entstehen, wie bereits oben gesagt, bei der Naphthaspaltung. Üblicherweise reichert man die Olefine weiter an, z.B. durch Extraktion des die Butene begleitenden Butadiens, oder durch Umwandlung des Isobutens in Methyl-tert.-butylether.

Ein typischer Vertreter solcher butenreicher Gemische ist das Raffinat II. Es weist, je nach Herstellungs- und Aufarbeitungsbedingungen, etwa folgende Zusammensetzung auf:

| Komponente | Raff. II Vol.-% |
|---|---|
| Isobutan | 3-8 |
| Isobuten | 0,5-1,5 |
| 1-Buten | 10-15 |
| n-Butan | 19-26 |
| 2-Buten (cis/trans) | 52-65 |

Selbstverständlich ist das neue Verfahren nicht auf den Einsatz von Ausgangsstoffen der beschriebenen Zusammensetzung beschränkt. Auch andere butenhaltige Gemische können zur Gewinnung von 2-Methylbutanal durch Hydroformylierung verwendet werden.

Die Hydroformylierung der Butene gehört zum Stand der Technik (vgl. Ullmann's Encyclopedia of Industrial Chemistry, 5. Auflage (1985), Band A 4, Seite 488). Sie kann nach den verschiedenen Ausführungsformen des Oxoverfahrens unter Einsatz von Kobalt- oder Rhodiumkatalysatoren erfolgen. Beispiele für geeignete Prozesse sind z.B. in der Chemiker Z. 96 (1972), S. 383 ff und in der DE-PS 2627354 beschrieben.

So kann die Hydroformylierung von Raffinat II unter Verwendung von Kobalt als Katalysator bei Temperaturen von 110 bis 180°C und Drücken von 20 bis 30 MPa erfolgen. Nach Abtrennen des Katalysators durch Behandeln des Reaktionsproduktes mit Wasser, Oxidationsmitteln, sauren Agenten und/oder anderen Additiven erhält man ein Gemisch, für das folgende Zusammensetzung typisch ist:

3

| | | |
|---|---|---|
| C$_4$-Kohlenwasserstoffe | 10 – 15 | Gew.-% |
| 2-Methylbutanal | 18 – 25 | Gew.-% |
| n-Pentanal | 40 – 50 | Gew.-% |
| 3-Methylbutanal | 0,5 – 1,5 | Gew.-% |
| Sonstige | 15 – 21 | Gew.-% |

Durch Destillation trennt man daraus die Kohlenwasserstoffe und eine aus C$_5$-Aldehyden bestehende Fraktion ab, die 80 bis 95 Gew.-% 2-Methylbutanal, 2 bis 5 Gew.-% 3-Methylbutanal und 5 bis 15 Gew.-% n-Pentanal enthält. Der Rückstand besteht im wesentlichen aus n-Pentanal.

Zur Umsetzung der C$_5$-Aldehydfraktion mit Formaldehyd wendet man je Mol n-Pentanal und 3-Methylbutanal (nachfolgend alpha-unverzweigte Aldehyde genannt) 1 bis 2 und insbesondere 1,1 bis 1,4 Mol Formaldehyd an. Der Formaldehyd wird bevorzugt als Lösung in Wasser eingesetzt, geeignet sind aber auch Lösungen in Alkoholen oder polymerisierter Formaldehyd, z.B. Paraformaldehyd. Im allgemeinen wird dem Reaktionsgemisch die gesamte Menge Formaldehyd zu Beginn der Umsetzung zugegeben. Es kann aber auch zweckmäßig sein, den Formaldehyd anteilsweise dem Aldehydgemisch zuzusetzen.

Die Umsetzung der alpha-unverzweigten Aldehyde mit Formaldehyd erfolgt unter dem Einfluß von Aminen als Katalysatoren. Bewährt haben sich sekundäre Amine der allgemeinen Formel R$^1$-NH-R$^2$, wobei R$^1$ und R$^2$ gleich oder verschieden sind und für Alkylreste mit je 1 bis 12, vorzugsweise 3 bis 5 Kohlenstoffatome stehen. Besonders geeignet ist Di-n-Butylamin. Vorteilhaft führt man die Reaktion in Gegenwart von Monocarbonsäuren mit 1 bis 10 Kohlenstoffatomen, oder Di- bzw. Polycarbonsäuren mit 2 bis 10 Kohlenstoffatomen durch. Bevorzugt werden Monocarbonsäuren mit 3 bis 5 Kohlenstoffatomen. Die Monocarbonsäuren und Polycarbonsäuren können aromatische, araliphatische und vorzugsweise aliphatische Carbonsäuren sein. Geeignet sind z.B. Essigsäure, Propionsäure, n-Buttersäure, iso-Buttersäure, Oxalsäure, Bernsteinsäure, Weinsäure. Bevorzugt setzt man n-Buttersäure ein. Je Mol alpha-unverzweigten Aldehyd wendet man 0,025 bis 0,3, vorteilhaft 0,1 bis 0,2 Mol Amin an. Je Mol alpha-unverzweigten Aldehyd werden 0,05 bis 0,5 und insbesondere 0,15 bis 0,3 Äquivalente Carbonsäuren eingesetzt.

Die Reaktion der alpha-unverzweigten Aldehyde mit Formaldehyd führt zur Bildung von alpha-Methylolaldehyden, die im allgemeinen unbeständig sind und unter Wasserabspaltung in die entsprechenden alpha-Methylenaldehyde (alpha-Alkylacroleine) übergehen. Der Siedepunkt dieser alpha-Methylenaldehyde unterscheidet sich deutlich von dem des ein Kohlenstoffatom weniger enthaltenden alpha-Methylaldehyds. Daher ist es ohne Schwierigkeiten möglich, die Aldehyde durch Destillation voneinander zu trennen.

Für das erfindungsgemäße Verfahren ist es charakteristisch, die Umsetzung des Aldehydgemischs mit Formaldehyd während der Destillation vorzunehmen. Zum Einsatz gelangen handelsübliche Destillationskolonnen mit Glasringen oder Metallwendeln als Füllkörper. Die Anzahl der theoretischen Böden beträgt in der Regel 9 bis 72 und bevorzugt 24 bis 36. Das Rücklaufverhältnis ist von der Art der eingesetzten Kolonne abhängig. Es kann z.B. bei einer Füllkörperkolonne mit 24 theoretischen Böden auf 5 Teile Rücklauf je Teil abgenommenes Kopfprodukt eingestellt werden.

Auch die Temperatur des Sumpfproduktes ist von der eingesetzen Kolonne abhängig. Verwendet man z.B. eine Füllkörperkolonne mit 24 Böden, so beträgt sie unter Normaldruck bis zu etwa 120°C.

Nach dem neuen Verfahren erhält man 2-Methylbutanal in 97 bis 99%iger Reinheit. Es ist lediglich durch geringe Anteile 3-Methylbutanal, Spuren n-Pentanal sowie inerte Bestandteile verunreinigt. Die Inerten stören bei der Weiterverarbeitung des 2-Methylbutanals z.B. zu Säure, Amin oder Alkohol nicht und sind aus dem Folgeprodukt des Aldehyds leicht zu entfernen.

Im folgenden Beispiel wird die Erfindung näher erläutert. Sie ist aber selbstverständlich nicht auf die hier beschriebene Ausführungsform beschränkt.

Beispiel

Raffinat II, das etwa 7 Vol.-% i-Butan, 25 Vol.-% n-Butan, 15 Vol.-% 1-Buten, 52 Vol.-% 2-Buten und 1 Vol.-% i-Buten enthält, wird bei etwa 150°C und einem Synthesegasdruck (CO : H$_2$ = 1 : 1) von etwa 22 MPa in Gegenwart eines Kobaltkatalystors hydroformyliert. Das Reaktionsprodukt wird entspannt und vom Katalysator befreit. Man erhält ein Gemisch, aus dem durch Destillation eine zwischen etwa 86 und etwa 93°C siedende Fraktion folgender Zusammensetzung erhalten wird (Angaben in Gew.-%) :

```
Vorlauf           :       1,2 %
3-Methylbutanal:          3,2 %
2-Methylbutanal:         83,4 %
n-Pentanal        :      11,2 %
Nachlauf          :       1,0 %
```

2000 g dieses Aldehydgemisches (3,33 mol alpha-unverzweigte Aldehyde) werden mit 399,4 g einer wäßrigen, 35 Gew.-% Formaldehyd enthaltenden Formalinlösung (4,66 mol), 64,5 g Di-n-butylamin (0,5 mol) und 59,0 g n-Buttersäure (0,67 mol) versetzt und diskontinuierlich fraktioniert destilliert. Die verwendete Füllkörperkolonne hat 24 theoretische Böden, das Rücklaufverhältnis wird auf 5 Teile Rücklauf je Teil abgenommenes Kopfprodukt eingestellt.

Bei einer Kopftemperatur von maximal 79°C und einer Sumpftemperatur von maximal 102°C erhält man eine Hauptfraktion, die aus 1582,3 g organischem Produkt und 285,6 g Wasserphase besteht.

Nach gaschromatographischer Analyse setzt sich das organische Produkt wie folgt zusammen (Angaben in Gew.-%):

```
Vorlauf           :       1,36 %
3-Methylbutanal   :       0,03 %
2-Methylbutanal   :      97,14 %
n-Pentanal        :          -
i-Propylacrolein:         0,05 %
Nachlauf          :       1,42 %
```

Es werden also 92,1 Gew.-% des eingesetzten 2-Methylbutanals aus der Hauptfraktion in nahezu isomerenfreier Form gewonnen. Weitere Anteile 2-Methylbutanal erhält man aus den höhersiedenden Fraktionen.

Das Produkt eignet sich unmittelbar zur Herstellung von Folgeverbindungen wie 2-Methylbutylamin, -butanol, -buttersäure. Durch einfache Redestillation wird es in 99%iger Reinheit gewonnen.

## Patentansprüche

1. Verfahren zur Gewinnung von 2-Methylbutanal aus den Gemischen der isomeren $C_5$-Aldehyde, insbesondere Gemischen, die bei der Hydroformylierung isomerer Butene entstehen, dadurch gekennzeichnet, daß man das $C_5$-Aldehydgemisch in Gegenwart von Formaldehyd und einem Aldolisierungskatalysator destilliert.

2. Verfahren nach Anspruch 1, dadurch gekennzeichnet, daß man je Mol im Aldehydgemisch enthaltenen n-Pentanal und 3-Methylbutanal 1 bis 2, insbesondere 1,1 bis 1,4 Mol Formaldehyd anwendet.

3. Verfahren nach Anspruch 2, dadurch gekennzeichnet, daß der Formaldehyd als Lösung in Wasser angewendet wird.

4. Verfahren nach einem oder mehreren der Ansprüche 1 bis 3, dadurch gekennzeichnet, daß man als Aldolisierungskatalysator sekundäre Amine der allgemeinen Formel $R^1$-NH-$R^2$ einsetzt, wobei $R^1$ und $R^2$ gleich oder verschieden sind und für Alkylreste mit je 1 bis 12, vorzugsweise 3 bis 5 Kohlenstoffatome stehen.

5. Verfahren nach Anspruch 4, dadurch gekennzeichnet, daß das sekundäre Amin Di-n-butylamin ist.

6. Verfahren nach einem oder mehreren der Ansprüche 1 bis 5, dadurch gekennzeichnet, daß man die Reaktion in Gegenwart einer Monocarbonsäure mit 1 bis 10 Kohlenstoffatomen oder Di- bzw. Polycarbonsäuren mit 2 bis 10 Kohlenstoffatomen durchführt.

7. Verfahren nach Anspruch 6, dadurch gekennzeichnet, daß man die Reaktion in Gegenwart einer Monocarbonsäure mit 3 bis 5 Kohlenstoffatomen, insbesondere n-Buttersäure, durchführt.

8. Verfahren nach einem oder mehreren der Ansprüche 1 bis 7, dadurch gekennzeichnet, daß man je Mol n-Pentanal und 3-Methylbutanal 0,025 bis 0,3, insbesondere 0,1 bis 0,2 Mol Amin anwendet.

9. Verfahren nach einem oder mehreren der Ansprüche 1 bis 8, dadurch gekennzeichnet, daß man je Mol n-Pentanal und 3-Methylbutanal 0,05 bis 0,5, insbesondere 0,15 bis 0,3 Äquivalente Carbonsäuren einsetzt.

**Claims**

1. A process for the recovery of 2-methylbutanal from the mixtures of isomeric $C_5$-aldehydes, in particular mixtures which are formed during the hydroformylation of isomeric butenes, characterised in that the $C_5$-aldehyde mixture is distilled in the presence of formaldehyde and an aldolisation catalyst.

2. A process according to claim 1, characterised in that 1 to 2, in particular 1.1 to 1.4 moles of formaldehyde are used per mole of n-pentanal and 3-methylbutanal contained in the aldehyde mixture.

3. A process according to claim 2, characterised in that the formaldehyde is used as a solution in water.

4. A process according to one or more of the claims 1 to 3, characterised in that secondary amines of the general formula $R^1$-NH-$R^2$ are used as the aldolisation catalyst, $R^1$ and $R^2$ being the same or different and standing for alkyl groups each having 1 to 12, preferably 3 to 5 carbon atoms.

5. A process according to claim 4, characterised in that the secondary amine is di-n-butylamine.

6. A process according to one or more of the claims 1 to 5, characterised in that the reaction is performed in the presence of a monocarboxylic acid having 1 to 10 carbon atoms or dicarboxylic or polycarboxylic acids having 2 to 10 carbon atoms.

7. A process according to claim 6, characterised in that the reaction is performed in the presence of a monocarboxylic acid having 3 to 5 carbon atoms, in particular n-butyric acid.

8. A process according to one or more of the claims 1 to 7, characterised in that 0.025 to 0.3, in particular 0.1 to 0.2 moles of amine are used per mole of n-pentanal and 3-methylbutanal.

9. A process according to one or more of the claims 1 to 8, characterised in that 0.05 to 0.5, in particular 0.15 to 0.3 equivalents of carboxylic acids are used per mole of n-pentanal and 3-methylbutanal.

**Revendications**

1. Procédé pour l'obtention de 2-méthylbutanal à partir des mélanges des aldéhydes en $C_5$ isomères, en particulier de mélanges qui se forment dans l'hydroformylation des butènes isomères, caractérisé en ce que l'on distille le mélange d'aldéhydes en $C_5$ en présence de formaldéhyde et d'un catalyseur d'aldolisation.

2. Procédé selon la revendication 1 caractérisé, en ce que l'on utilise 1 à 2 mol de formaldéhyde, en particulier 1,1 à 1,4 mol, par mole de n-pentanal et de 3-méthylbutanal contenus dans le mélange d'aldéhydes.

3. Procédé selon la revendication 2, caractérisé en ce que l'on utilise le formaldéhyde en solution dans l'eau.

4. Procédé selon une ou plusieurs des revendications 1 à 3, caractérisé en ce que l'on utilise comme catalyseurs d'aldolisation des amines secondaires de formule générale $R^1$-NH-$R^2$, dans laquelle $R^1$ et $R^2$ sont identiques ou différents et représentent des restes alkyles chacun en $C_1$-$C_{12}$, de préférence en $C_3$-$C_5$.

5. Procédé selon la revendication 4, caractérisé en ce que l'amine secondaire est la di-n-butylamine.

6. Procédé selon une ou plusieurs des revendications 1 à 5, caractérisé en ce que l'on met en oeuvre la réaction en présence d'un acide monocarboxylique en $C_1$-$C_{10}$ ou d'acides di- ou polycarboxyliques en $C_2$-$C_{10}$.

7. Procédé selon la revendication 6, caractérisé en ce que l'on met en oeuvre la réaction en présence d'un acide monocarboxylique en $C_3$-$C_5$, en particulier l'acide n-butyrique.

8. Procédé selon une ou plusieurs des revendications 1 à 7, caractérisé en ce que l'on utilise 0,025 à 0,3 mol, en particulier 0,1 à 0,2 mol, d'amine par mole de n-pentanal et de 3-méthylbutanal.

9. Procédé selon une ou plusieurs des revendications 1 à 8, caractérisé en ce que l'on utilise 0,05 à 5 équivalents d'acides carboxyliques, en particulier 0,15 à 0,3 équivalent, par mole de n-pentanal et de 3-méthylbutanal.